# EUROPÄISCHE PATENTANMELDUNG

(11) **EP 2 792 735 A1**
(43) Veröffentlichungstag der Anmeldung: **22.10.2014**
(21) Anmeldenummer: 13163922.1
(22) Anmeldetag: 16.04.2013
(51) Int. Cl.: C11B 3/00, C12P 7/64

(54) **Verfahren zur Verbesserung der wässrigen enzymatischen Entschleimung von Pflanzenölen**

(71) Anmelder: Clariant Produkte (Deutschland) GmbH, 65929 Frankfurt am Main (DE)
(72) Erfinder: Suck, Kirstin, 80939 München (DE); Sohling, Ulrich, 85356 Freising (DE); Ruf, Friedrich, 84184 Tiefenbach-Ast (DE); Bubenheim, Paul, 22359 Hamburg (DE)

(57) **Zusammenfassung**

Die Erfindung betrifft ein Verfahren zur Entschleimung von Pflanzenölen oder zur Verringerung des Ölgehalts im Pflanzenölschleim unter Verwendung mindestens eines Glykosidspaltenden Enzyms, wobei das mindestens eine Glykosidspaltende Enzym keine Phospholipase- und keine Acyltransferase-Aktivität aufweist bzw. keine Phospholipase und keine Acyltransferase im erfindungsgemäßen Enzym vorhanden sind.

## Beschreibung

Die Erfindung betrifft ein Verfahren zur enzymatischen Entschleimung von Triglyceriden, insbesondere von rohen Pflanzenölen, wobei die Phosphatide unverändert bleiben. Ferner ist ein Verfahren zur Verringerung des Ölgehalts in Pflanzenölschleim bzw. die Gewinnung von Lecithin aus Pflanzenölen, insbesondere aus Raps- und Sojaöl, Gegenstand dieser Erfindung.

Rohe Pflanzenöle enthalten Phosphatide, eiweiß- und kohlenhydrathaltige Stoffe, pflanzliche Schleimstoffe sowie kolloidale Verbindungen, die die Haltbarkeit des Öls stark herabsetzen. Diese Stoffe müssen daher entfernt werden.

Bei der Raffination von Pflanzenölen werden die unerwünschten Begleitstoffe entfernt. Man unterscheidet zwischen chemischer und physikalischer Raffination. Die chemische Raffination besteht aus den Prozessen 1. Entschleimung, 2. Neutralisation, 3. Bleichung, 4. Desodorierung. Bei der Entschleimung werden Phospholipide ("Schleimstoffe") und Metallionen aus dem Öl entfernt. Die Neutralisation dient zur Extraktion der Fettsäuren. Bei der Bleichung werden die Farbstoffe, weitere Metallionen und restliche Schleimstoffe entfernt. Bei der Desodorierung handelt es sich um eine Wasserdampfdestillation, bei der weitere Verbindungen entfernt werden, die den Geruch und den Geschmack des Öls beeinträchtigen. Bei der physikalischen Raffination wird die Entsäuerung zusammen mit der Desodorierung am Ende des Raffinationsprozesses durchgeführt.

Die Entschleimung der Öle kann durch Extraktion der Phospholipide mit Wasser oder einer wässrigen Lösung einer Säure erfolgen, die Ca²⁺- und Mg²⁺-Ionen komplexiert, wie z. B. Zitronensäure oder Phosphorsäure. Häufig führt man hierbei zunächst eine wässrige, sogenannte Vorentschleimung durch, mit der die wasserlöslichen Phospholipide entfernt werden. Man spricht hierbei von hydratisierbaren Phospholipiden.

### Definitionen

Unter dem Begriff **Triglyceride** werden dreifache Ester des Glycerins mit Fettsäuren verstanden, welche den Hauptbestandteil von natürlichen Fetten und Ölen darstellen, sei es pflanzlichen oder tierischen Ursprungs. Triglyceride umfassen pflanzliche oder tierische Fette und Öle, und deren Mischungen sowohl untereinander als auch mit synthetischen bzw. modifizierten Fetten und Ölen.

Unter dem Begriff **Pflanzenöl** wird hierbei jedes Öl pflanzlichen Ursprungs verstanden. Bevorzugte Öle sind im Sinne der vorliegenden Erfindung Sojaöl, Rapsöl, Sonnenblumenöl, Olivenöl, Palmöl, Jatrophaöl, Leindotteröl, oder Baumwollsaatöl. Überdies umfasst das Pflanzenöl im Sinne der Erfindung Mischungen verschiedener Pflanzenöle untereinander, als auch die Mischung von Pflanzenöl mit tierischen und/oder synthetischen bzw. modifizierten Fetten und Ölen.

Die Bezeichnung **"roh"** bezieht sich dabei darauf, dass das Öl noch keinem Entschleimungs-, Neutralisations-, Bleichungs-und/oder Desodorierungsschritt unterzogen wurde. Es ist im Rahmen des erfindungsgemäßen Verfahrens auch möglich, dass eine Mischung mehrerer Rohöle eingesetzt wird oder vorbehandelte, z.B. vorentschleimte und/oder vorkonditionierte Öle mit den Enzymen behandelt werden.

Unter **Schleimphase** / Schleimstoffe / Pflanzenölschleim versteht man hier im folgenden Text die gesamte Gruppe dieser Stoffe, die nach Behandlung mit einer säurehaltigen und/oder wässrigen Lösung aus dem Öl als schwere Phase ausfallen (Michael Bokisch: Fats and Oils Handbook, AOCS Press, Champaign, Illinois, 1998, Seite 428-444. Die Verwendung dieses Pflanzenölschleims als Einsatzmaterial ist insbesondere für die Gewinnung von Lecithin von Bedeutung, da Lecithin ein wesentlicher Bestandteil des Pflanzenölschleims ist.

Unter dem Begriff **"Verringerung des Ölgehalts im Pflanzenölschleim"** versteht man die Abtrennung des Öls aus dem eingesetzten Pflanzenölschleim, welcher dadurch "entölt" wird. Je nach Betrachtungsweise steht die Gewinnung von Öl und/oder die Gewinnung der Lecithin-haltigen Schleimphase im Vordergrund.

Unter dem Begriff **"Vorentschleimung"** bzw. "Naßentschleimung" wird eine Behandlung des Rohöls mit Wasser oder einer wässrigen Säurelösung verstanden, um wasserlösliche Phospholipide weitestgehend aus dem Öl zu entfernen. Auch kann im Rahmen einer Vor- bzw. Naßentschleimung nach der Säurezugabe ggf. eine Zugabe von Alkali erfolgen, um die Säure zu neutralisieren. Vor der Enzymzugabe erfolgt die Abtrennung der wässrigen Phase. Nach einer Vorentschleimung wird der Phosphorgehalt im Rohöl von ca. 500-1500 ppm, z.B. für Soja und Raps auf unter 200 ppm im vorentschleimten Öl gesenkt. Durch die Vorentschleimung kann z.B. Lecithin aus der entstandenen Schleimphase gewonnen werden bzw. die Schleimphase als Futtermittel aufgearbeitet werden. Der Nachteil der Abtrennung der wässrigen Phase bzw. der Senkung des Phosphorgehaltes ist jedoch ein Ausbeuteverlust bezüglich des Öls. Die in die wässrige Phase übergehenden Phosphatide wirken emulgierend und führen dazu, dass ein Teil des Öls in der wässrigen Phase emulgiert und mit dieser abgetrennt wird. Anschließend kann das Öl enzymatisch weiterbehandelt werden (,wobei die Enzyme in einem weiteren Schritt abgetrennt werden müssen).

Unter dem Begriff **"Vorkonditionierung"** des Öls wird in dieser Anmeldung die Zugabe von Wasser bzw. einer wässrigen Säurelösung zu dem unbehandelten Öl verstanden. Anschließend wird durch Zugabe von Alkali, z. B. Natronlauge, ein pH-Wert eingestellt, bei dem die folgende enzymatische Reaktion stattfindet. Idealerweise wird der für die Enzymreaktion optimale pH-Wert eingestellt. Anschließend erfolgt jedoch keine Abtrennung der wässrigen Phase, sondern unmittelbar die Zugabe der Enzyme. Die vorhandenden Schleimstoffe verbleiben also vorerst im Öl bzw. in der Emulsion. Die Abtrennung der wässrigen Phase und damit der Enzyme erfolgt erst nach Einwirkung der Enzyme auf das (ggf. vorkonditionierte) Rohöl.

### Stand der Technik

Die Thematik der hydratisierbaren und nicht-hydratisierbaren Phospholipide ist beispielsweise beschrieben in Nielsen, K., Composition of difficultly extractable soy bean phosphatides, J. Am. Oil. Chem. Soc. 1960, 37, 217-219 und A.J. Dijkstra, Enzymatic degumming, Eur. J. Lipid Sci. Technol. 2010, 112, 1178-1189. Dabei handelt es sich insbesondere um Phosphatidyl-Cholin und Phosphatidyl-Inositol. Die Behandlung mit verdünnten wässrigen Calcium- und Magnesium-komplexierenden Säuren, wie z. B. Zitronensäure oder Phosphorsäure, führt nach dem Stand der Technik dazu, dass nicht-hydratisierbare Phospholipide in hydratisierbare Phospholipide überführt werden. Man geht davon aus, dass der Mechanismus dieser Reaktion darauf beruht, dass Calciumionen, die verschiedene Phospholipidmoleküle, wie z.B. Phosphatidsäuren an den Phosphatgruppen, überbrücken und stabilisieren, aus dem Öl entfernt werden. Dies führt zur verbesserten Extraktion dieser Phospholipide mit Wasser. Zwar wird durch die wässrige Vorentschleimung und die Behandlung mit wässrigen Säuren bei einigen Ölen, wie z. B. Palmöl, eine hinreichend gute Entschleimung erreicht, für andere Pflanzenöltypen, wie z. B. Canolaöl, Rapsöl oder Sojaöl, führen diese beiden Extraktionsschritte sehr oft zu einer ungenügenden Reduktion der Schleimstoffe. Üblicherweise wünscht man sich hierbei eine Reduktion des Phosphorgehaltes auf 10 oder weniger ppm Phosphor im Öl für Nahrungsmittelanwendungen (nach dem Stand der Technik bestimmt durch ICP/AES-Analyse des Öls). Stärkere Anforderungen werden an den Phosphorgehalt des Öls dann gestellt, wenn die Öle beispielsweise zur Herstellung von Biodiesel eingesetzt werden. Dort ist nach der EU-Norm der Phosphorgehalt des Biodiesels auf 5 ppm begrenzt und es ist zweckmäßig, die Phosphorreduktion bereits ölseitig durchzuführen. Weiterhin ist ein besonders niedriger Phosphorgehalt, d. h. ein Phosphorgehalt, der so niedrig wie möglich ist und möglichst 0 beträgt, dann erforderlich, wenn man die Öle in weiteren Behandlungsschritten entweder für den Nahrungsmitteleinsatz hydriert, d. h. ungesättigte in gesättigte Fettsäuren überführt, oder wenn man nach dem NExBTL-Prozess der Firma Neste eine Hydrierung so durchführt, dass als Endprodukt Alkane, das heißt ein konventioneller Biodieseltreibstoff, jedoch hergestellt aus Pflanzenöl, erhalten wird. Diese Prozesse stellen, wie vorher angeführt, extrem hohe Anforderungen an einen niedrigen Phosphorgehalt im Öl, und der Einsatz solcher Prozesse wird in dem Maße zunehmen wie Pflanzenöle als Rohstoffe für die chemische Industrie eingesetzt werden.

Eine weitere Variante stellt das sog. "Caustic Refining" dar. Dieses Verfahren wird eingesetzt, um möglichst alle Phospholipide zusammen mit freien Fettsäuren aus dem Öl zu entfernen. Dieser Prozess ist beispielsweise in der WO 08/094847 beschrieben. Bei diesem Verfahren wird das rohe oder Wasser-vorentschleimte Öl zunächst mit geringen Mengen an Zitronensäure oder Phosphorsäure vermischt und intensiv gerührt. Hierbei werden, wie vorher schon erläutert, Salze von nicht hydratisierbaren Phospholipiden stärker hydratisierbar gemacht. Dann wird verdünnte Natronlauge hinzugefügt, wobei die Menge so berechnet wird, dass man einen geringen Überschuss gegenüber der zur Neutralisation der freien Fettsäure benötigten Menge erhält. Dadurch werden die Fettsäuresalze gebildet. Durch Absetzen und nachfolgende Zentrifugation wird die Mischung dann aufgetrennt und man erhält eine wässrige Fettsäurelösung als Rückstand, in der sich auch die Phospholipide befinden. Das Öl wird dann nachfolgend nochmals mit enthärtetem Wasser gewaschen. Die NaOH-Behandlung hat den Nachteil, dass teilweise auch Verseifung des Öles eintritt, wodurch dessen Ausbeute erniedrigt wird.

Nach dem Stand der Technik lässt sich eine weitere Reduktion des Phosphorgehaltes im Öl dadurch erzielen, dass man entweder eine Adsorbensbehandlung mit einer Bleicherde oder einem speziellen Kieselgel durchführt oder die Pflanzenöle enzymatisch entschleimt. Die Adsorbensbehandlung weist den Nachteil auf, dass nach der Adsorbensbehandlung Pflanzenöl am Adsorbens verbleibt, welches die Ölausbeute des gesamten Raffinationsprozesses vermindert. Außerdem stellt die gebrauchte Bleicherde Abfall dar, für den Entsorgungsmöglichkeiten gefunden werden müssen.

Ein weiterer Nachteil der konventionellen Ölentschleimungsprozesse liegt darin, dass sowohl die wässrige Vorentschleimung als auch die Behandlung mit wässrigen Säuren zu Ölverlusten führen, die dadurch verursacht werden, dass die in das Wasser überführten Phospholipide Emulgatoren darstellen, die einen geringen Teil des Pflanzenöls in der wässrigen Phase emulgieren, wodurch Pflanzenöl verloren geht. Diese Verluste können im Bereich weniger Prozente bezogen auf das ursprünglich eingesetzte Rohöl liegen. Als Faustregel gilt, dass mit je zwei Molekülen Phopholipid etwa ein Triglyceridmolekül emulgiert wird (beschrieben in WO 08/094847). Berücksichtigt man jedoch die Menge des weltweit zu raffinierenden Öls, nämlich 43 Mio t Sojaöl und 23,5 Mio t Rapsöl jeweils pro Jahr, so stellt der Anteil des Öls, der aufgrund dieser Emulsionswirkung ins Wasser übertritt und somit verloren geht, einen beträchtlichen Verlust dar.

Die sogenannte enzymatische Entschleimung vermeidet mehrere Nachteile der bestehenden Verfahren bzw. verbessert die Extraktionsverfahren weiter. So entsteht kein zusätzlicher Abfall wie beim Einsatz von Adsorbentien und es wurde gezeigt, dass bei der enzymatischen Entschleimung die Ölverluste weiter verringert werden können.

Die sogenannte enzymatische Entschleimung wird im Stand der Technik durch den Einsatz von Phospholipasen, insbesondere Phospholipase A1 und A2, B oder Phospholipase C oder einer Kombination von Phospholipasen herbeigeführt. Phospholipasen sind Enzyme, die zur Gruppe der Hydrolasen gehören, die die Esterbindung von Phospholipiden hydrolysieren.

Eine weitere Variante der enzymatischen Ölentschleimung stellt die enzymatische Behandlung der abgetrennten Schleimphase dar, nachdem das Öl nach konventionellen Verfahren wie z.B. mit Wasser und/oder Zitronensäure entschleimt wurde. Durch diese Behandlung ist es möglich, einen Teil des in der Schleimphase emulgierten Pflanzenöls wiederzugewinnen.

Betrachtet man die Gewinnung von Lecithin für den Einsatz in Nahrungsmitteln oder in Tierfuttermitteln, so wird dieses aus der wässrigen Lösung gewonnen, welche aus der wässrigen Vorentschleimung des Pflanzenöles erhalten wird. Hierbei wird über Dünnschichtverdampfer das Wasser entfernt. Des Weiteren ist es für viele Anwendungen wünschenswert, den Ölgehalt von ca. 30 % noch stark zu verringern, in der Regel auf unter 5 %.

Nach dem Stand der Technik wird die Entölung des Rohlecithins im Wesentlichen durch eine Acetonextraktion erzielt, wie sie beispielsweise in der WO 94/01004 beschrieben ist. In der WO 94/01004 wird als Alternative die Extraktion des Öls mit überkritischem CO₂ diskutiert. Die Entölung des rohen Lecithins ist für die meisten Anwendungen erforderlich, wenn das Lecithin als Emulgator eingesetzt werden soll, da das vorhandene Öl die Emulgierfähigkeit verringert und auch den Aktivgehalt des Lecithins herabsetzt.

Auch beim Einsatz als Futtermittelkomponente ist es in einigen Fällen wünschenswert, das rohe Lecithin zu entölen. Entsprechende Qualitäten von Futtermittellecithin sind auf dem Markt erhältlich, wie z. B. das Produkt Bergapur von der Firma Berg und Schmidt Hamburg, bei dem es sich um ein entöltes Lecithin mit einem Lecithingehalt von 96% handelt und das als Futtermittelkomponente für die Aquakultur, Geflügel, Schweine und weitere Tierarten eingesetzt wird. Analoge nicht entölte handelsübliche Produkte für einen Tierfuttereinsatz weisen dagegen lediglich Lecithingehalte von 50 - 60 % auf.

In der Regel müssen alle Pflanzenöle entschleimt werden und der Gehalt an Ionen wie z. B. Phosphor, Magnesium und Calcium unter gewisse Grenzwerte gebracht werden. Raps-, Sonnenblumen-oder Sojaöl sind wegen des hohen Phospholipid-Gehalts zum Teil schwer zu entschleimen. Im Falle des Sojaöls ist sowohl das (entschleimte) Öl als auch das gewonnene Soja-Lecithin zur Verwendung als Lebensmittel bzw. Lebensmittel-Hilfsstoff von Interesse.

Die enzymatischen Ölentschleimungs-Verfahren auf der Basis von Phospholipasen haben den Vorteil, dass durch die Verringerung der Emulgierkapazität des Lecithins die Ölausbeute höher ist als bei den konventionellen Entschleimungsverfahren. Führt man die enzymatische Ölentschleimung direkt mit dem Rohöl durch, erhält man in der wässrigen Phase - bedingt durch das Verfahren - ein Gemisch von Phospholipiden und Lysophospholipiden, d. h. aus solchen Phospholipiden, bei denen Fettsäuren abgespalten wurden. Dieses Gemisch weist aufgrund des Gehaltes an Lysolecithin ein anderes Emulgierverhalten als die eines Lecithins auf, wie es durch den Standardprozess der wässrigen Vorentschleimung gewonnen wird. Emulgatoren werden durch den sogenannten HLB Wert klassifiziert. Phospholipide haben in der Regel einen HLB Wert um 5, während der HLB Wert von Lysophosphatiden in der Regel bei 15-18 liegt. Daraus ergeben sich völlig unterschiedliche Einsatzmöglichkeiten. Emulgatoren mit einem HLB-Wert von 3-6 werden in der Regel zur Herstellung von Wasser- in Öl-Emulsionen eingesetzt, während solche mit HLB Werten von 8- 18 für die Herstellung von Öl- in Wasser-Emulsionen verwendet werden; (Surfactants and Polymers in Aqueous solutions; B. Jönsson, B. Lindman, K. Holmberg, B. Kronberg; p. 251, Chapter 17, Use of Surfactants as Emulsifiers, Wiley 1998).

Die Lysophospholipide müssen nun von den restlichen Phospholipiden abgetrennt werden, wenn sie speziellen Emulgatoranwendungen, wie z.B. für Lebensmittel, zugeführt werden sollen; alternativ bzw. ohne Abtrennung der Lysophospholipide kann das Lecithin lediglich als Futtermittelqualität eingesetzt werden.

### Aufgabe der Erfindung

Die vorliegende Erfindung hat sich die Aufgabe gestellt, die Entschleimung von Triglyceriden so zu verbessern, dass die Phospholipide in ihrer chemischen Struktur und folglich auch in ihrem Emulgierverhalten unverändert bleiben, aber andererseits weniger Öl im abgetrennten Schleim verbleibt. Ein Ziel der Erfindung ist daher die Erhöhung der Ölausbeute. Eine weitere Aufgabe der Erfindung ist die Bereitstellung eines Verfahrens zur Gewinnung von Lecithin aus Triglyceriden, insbesondere aus rohem Soja-, Sonnenblumen- oder Rapsöl, mit hoher Ausbeute und ohne chemische Veränderung des Lecithins, wobei der Gehalt an Öl im gewonnen Lecithin möglichst gering ist, oder mit anderen Worten, die Entölung des Lecithins bzw. die Verringerung des Ölgehalts im Pflanzenölschleim.

### Lösung der Aufgabe

Die Aufgabe wird durch ein Verfahren zur enzymatischen Entschleimung von Triglyceriden oder zur Verringerung des Ölgehaltes im Pflanzenölschleim, der bei der Ölentschleimung anfällt, gelöst, welches Verfahren die folgende Schritte umfasst:

Zunächst wird das Triglycerid oder der Pflanzenölschleim, der bei der Ölentschleimung anfällt, in Schritt a) mit einer Zusammensetzung in Kontakt gebracht, die mindestens ein Glykosid-spaltendes Enzym umfasst, wobei das mindestens eine Glykosid-spaltende Enzym keine Phospholipase- und keine Acyltransferase-Hauptaktivität aufweist bzw. keine Phospholipase und keine Acyltransferase in der Zusammensetzung vorhanden sind.

Anschließend werden, wenn Triglyceride aus Ausgangsmaterial verwendet werden, die Schleimstoffe in Schritt b1) von den Triglyceriden abgetrennt. Bevorzugt werden rohe Pflanzenöle als Ausgangsmaterial des Verfahrens eingesetzt.

Alternativ kann anstelle der Triglyceride auch Pflanzenölschleim eingesetzt werden, der bei Entschleimung von Pflanzenölen anfällt, sei es durch eine Entschleimung nach herkömmlichen oder dem erfindungsgemäßen Verfahren. Der Pflanzenölschleim wird gemäß Schritt a) mit dem Glykosid-spaltenden Enzym in Kontakt gebracht und anschließend nach Schritt b2), der analog zu b1) durchgeführt wird, in eine wässrige, Lecithin-haltige Phase und eine Öl-haltige Phase getrennt. Die Schleimphase bzw. der Pflanzenölschleim wird insbesondere bei der Gewinnung von Lecithin eingesetzt.

Das Glykosid-spaltende Enzym weist, wenn überhaupt, nur eine durch Verunreinigung entstandene, Phospholipid-spaltende Nebenaktivität auf, die dadurch definiert ist, dass der Gehalt an freien Fettsäuren während einer Reaktionszeit von 4 h nicht mehr als relativ 10 %, bevorzugt nicht mehr als relativ 8 % ansteigt. Diese Werte beziehen sich auf die relative Zunahme der Fettsäure-Konzentration, die als Anteil der freien Fettsäuren (FFA), ausgedrückt als Ölsäure, bezogen auf die gesamten Fettsäuren, definiert ist. Die Bestimmung der freien Fettsäuren (FFA) ist im Abschnitt Methoden beschrieben.

Ein Triglycerid im Sinne der vorliegenden Erfindung ist vorzugsweise ein Pflanzenöl oder ein Gemisch aus einem pflanzlichen und einem tierischen Öl.

Insbesondere weist das mindestens eine Glykosid-spaltende Enzym eine Substratspezifität dergestalt auf, dass es α(1-4)glykosidische, α(1-2)glykosidische, α(1-6)glykosidische, β(1-2)glykosidische, β(1-3)glykosidische, β(1-4)glykosidische und/oder β(1-6)glykosidische Bindungen spaltet. Bevorzugt werden α(1-4)glykosidische Bindungen gespalten.

In einer Ausführungsform wird das mindestens eine Glykosid-spaltende Enzym aus Amylasen, Amyloglucosidasen, Isoamylasen, Glucoamylasen, Glucosidasen, Galactosidasen, Glucanasen, Pullulanasen, Arabinasen, Laminaranasen, Pektolyasen, Mannanasen, Dextranasen, Pectinasen, Cellulasen, Cellobiasen, und Xylanasen ausgewählt.

Insbesondere ist die Amylase eine alpha-Amylase und bevorzugt eine alpha-Amylase, welche spezifisch α(1-4)glykosidische Bindungen spaltet.

Besonders bevorzugt stammt die alpha-Amylase aus folgenden Spezies: Bacillus sp., Bacillus subtilis, Bacillus licheniformis, Bacillus megaterium, Bacillus amyloliquefaciens, Bacillus stearothermophilus, Pseudomonas aeroginosus, Pseudomonas fluorescens, Aspergillus oryzae, oder Aspergillus niger, insbesondere auch Bacillus subtilis und Aspergillus oryzae.

In einer bevorzugten Ausführungsform wird nur alpha-Amylase als Enzym eingesetzt, insbesondere alpha-Amylase aus Bacillus subtilis und Aspergillus oryzae.

Gemäß einer Ausführungsform kann das eine oder mehrere der eingesetzten Glykosid-spaltenden Enzyme in geträgerter Form vorliegen.

Die bevorzugt eingesetzten Öle der vorliegenden Erfindung sind Sojaöl, Rapsöl, Sonnenblumenöl, Olivenöl, Palmöl, Jatrophaöl, Leindotteröl, oder Baumwollsaatöl, insbesondere Sojaöl, Rapsöl oder Sonnenblumenöl. Bevorzugt werden die genannten Öle im rohen Zustand im erfindungsgemäßen Verfahren gemäß den Schritten a) und b1) eingesetzt.

Alternativ kann - anstelle des Pflanzenöls selbst - auch ein Pflanzenölschleim in den Schritten a) und b2) eingesetzt werden, der durch Abtrennung aus den vorstehend genannten Ölen erhalten wurde. Dadurch kann das in der Schleimphase enthaltene Öl zurückzugewonnen und überdies das im Schleim enthaltene Lecithin entölt werden.

Eine Ausführungsform betrifft die Verwendung eines Glykosid-spaltenden Enzyms zur Erhöhung der Ölausbeute bei der Durchführung einer wässrigen Öl-Entschleimung, und ferner zur Entölung von Lecithin.

### Vorteile der Erfindung

Aufgrund des Umstands, dass keine Phospholipase oder Acyltransferase-Aktivität während des EntschleimungsVerfahrens vorhanden ist, bleiben die abgetrennten Phospholipide in ihrer chemischen Struktur und ihrem Emulgierverfahren unverändert. Durch das erfindungsgemäße Verfahren steigt die Ölausbeute des Prozesses, und der Ölgehalt des Schleimes sowie das Schleimvolumen werden verringert. Da die Konzentration des Öls in der Schleimphase verringert wird, sinkt überdies der Aufwand für eine nachträgliche Entölung des Lecithins für Spezialanwendungen. Insbesondere sinkt die für die Entölung des Lecithins notwendige Menge des Acetons (vgl. US 4,803,016). Ein weiterer Vorteil der Erfindung ist, dass der Gehalt des im Öl verbleibenden Lecithins möglichst gering ist, die Abtrennung des Lecithins aus dem Öl also in hoher Ausbeute erfolgt.

### Genaue Beschreibung der Erfindung

In diesem Zusammenhang wurde nun erstaunlicherweise gefunden, dass die gestellte Aufgabe dadurch lösbar ist, dass man bei der Entschleimung von Triglyceriden ein Enzym zusetzt, welches nicht auf die Phospholipide einwirkt, wie z. B. Phospholipasen oder Acyltransferasen, sondern lediglich Nebenkomponenten des Schleimes, wie z.B. oligomere oder polymere Kohlenhydrate oder Glykoside von Phenolsäuren oder Glycolipide enzymatisch spaltet. Es kann angenommen werden, dass durch die Spaltung dieser Verunreinigungen die Phasentrennung verbessert und weniger Öl in dem wässrigen Schleim festgehalten wird.

Bei den erfindungsgemäßen Enzymen handelt es sich um ein oder mehrere Enzyme aus der Gruppe der Glykosidasen.

Bei den Enzymen, die für das erfindungsgemäße Verfahren eingesetzt werden können, handelt es sich außerdem um ein oder mehrere Enzyme, die keine Phospholipid-spaltenden Enzyme darstellen.

Bei dem "Phospholipid-spaltenden Enzym" kann es sich um eine Phospholipase handeln, die in der Lage ist, entweder einen Fettsäurerest oder einen Phosphatidylrest oder eine Kopfgruppe von einem Phospholipid abzuspalten. Beispiele sind die Phospholipase A1, Phospholipase A2, Phospholipase C, Phospholipase B, Phospholipase D oder Mischungen aus Phospholipasen. Des Weiteren kann es sich auch um eine sogenannte Acyltransferase handeln, bei der die Abspaltung des Fettsäurerests mit einer Übertragung dieses Restes, gefolgt von einer Esterbildung, mit einem freien Sterol in der Ölphase verbunden ist.

Ebenso ist es bevorzugt, dass vorliegend keine Phosphatasen, d.h. Enzyme mit Phosphatase als Hauptaktivität, bzw. keine Glykosid-spaltenden Enzyme mit Phosphatase als Nebenaktivität eingesetzt werden. Unter dem Begriff Phosphatase werden eine Gruppe von Enzymen verstanden, die aus Phosphorsäureestern oder Polyphosphaten Phosphorsäure abspalten.

Die vorstehend beschriebenen Klassen von Enzymen, Phospholipasen, Acyltransferasen und Phosphatasen, werden in der vorliegenden Erfindung vorzugsweise nicht eingesetzt - sei es als Enzyme mit Hauptaktivität oder als Nebenaktivität anderer Enzyme - um die Phospholipide, die aus den Ölen abgetrennt werden, in ihrer chemischen Struktur und ihrem Emulgierverhalten nicht zu verändern.

Um das Lecithin, welches nach dem erfindungsgemäßen Verfahren aus pflanzlichen Ölen abgetrennt wird, chemisch unverändert zu erhalten bzw. seine Emulgiereigenschaften nicht zu verändern, ist es notwendig, dass die erfindungsgemäß verwendeten Enzyme keine Phospholipase- und keine Acyltransferase-Aktivität aufweisen, jeweils betrachtet aus der Perspektive einer möglichen Nebenaktivität der erfindungsgemäß eingesetzten Glykosid-spaltenden Enzyme.

Ebenso wenig sollen eine Phospholipase und eine Acyltransferase in der Zusammensetzung zur enzymatischen Entschleimung von pflanzlichen Ölen nicht vorhanden sein, jeweils als Enzym mit einer entsprechenden Hauptaktivität.

Bezüglich der erfindungsgemäßen Glykosid-spaltenden Enzyme sind solche bevorzugt, die α(1-4)glykosidische, α(1-2)glykosidische, α(1-6)glykosidische,β(1-2)glykosidische, β(1-3)glykosidische, β(1-4)glykosidische und/oder β(1-6)glykosidische Bindungen spalten, z.B. Amylasen, Amyloglucosidasen, Isoamylasen, Glucoamylasen, Glucosidasen, Galactosidasen, Glucanasen, Pullulanasen, Arabinasen, Laminaranasen, Pektolyasen, Mannanasen, Dextranasen, Pectinasen, Cellulasen, Cellobiasen, und Xylanasen. Dabei kann auch eine Kombination aus zwei oder mehr der vorgenannten Glykosid-spaltenden Enzyme eingesetzt werden.

Die Enzyme können dabei von einem beliebigen Organismus (z.B. auch isoliert aus einem thermophilen Organismus) oder einer synthetischen Quelle stammen. Es ist im Rahmen der vorliegenden Erfindung auch möglich, dass Enzyme gleicher Art eingesetzt werden, die jedoch aus unterschiedlichen Quellen bzw. Spezies stammen. Ebenso sind rekombinant hergestellte, chimäre Fusionsproteine aus zwei oder mehreren verschiedenen Spezies mit enzymatischer Aktivität umfasst.

Es sind ferner Amylasen, insbesondere α-Amylasen, β-Amylasen, γ-Amylasen und Isoamylasen, sowie Mannanasen bevorzugt.

Bezüglich der Amylasen und Mannanasen sind solche aus Bacillus bzw. Pseudomonas bzw. fungalen Spezies oder aus Pankreas (Säugetier) bevorzugt, insbesondere solche aus Bacillus sp., Bacillus subtilis, Bacillus licheniformis, Bacillus megaterium, Bacillus amyloliquefaciens, Bacillus stearothermophilus, Pseudomonas aeroginosus, Pseudomonas fluorescens, Aspergillus oryzae, Aspergillus niger oder Trichoderma reesei. Im Falle der Mannanase ist eine solche aus Trichoderma reesei besonders bevorzugt.

Für die Entschleimung von Sojaöl ist insbesondere eine α-Amylase aus Bacillus-Species bevorzugt. Für die Entschleimung von Rapsöl ist insbesondere eine **α**-Amylase aus Bacillus-Spezies oder Aspergillus-Spezies bevorzugt, insbesondere Bacillus subtilis bzw. Aspergillus oryzae.

Der Zweck des erfindungsgemäßen Verfahrens liegt in der Gewinnung von Öl, das möglichst von Schleimstoffen bzw. Lecithin befreit ist, und aus umgekehrter Perspektive in der Gewinnung von Lecithin, das möglichst von Öl befreit ist und einen wesentlichen Bestandteil der Schleimphase darstellt.

Es können **Triglyceride,** vorzugsweise rohe oder unbehandelte Triglyceride, als Ausgangsmaterial eingesetzt werden, die mit dem Glykosid-spaltenden Enzym in Kontakt gebracht werden (Schritt a) und anschließend in Schleimstoffe und (entschleimte) Triglyceride getrennt werden; (Schritt b1). In diesem Fall wird das Glykosid-spaltende Enzym **vor** dem Trennschritt b1) zugegeben.

Alternativ zu den Triglyceriden kann ein **Pflanzenölschleim,** der z.B. durch ein herkömmliches Entschleimungs-Verfahren, wie z.B. durch Behandlung mit Wasser oder wässriger Säure, erhalten wurde, mit dem Glykosid-spaltenden Enzym in Kontakt gebracht werden (Schritt a) und anschließend in eine wässrige Lecithin-haltige Phase und eine Ölphase aufgetrennt werden; (Schritt b2). In Fall der Abtrennung des Pflanzenölschleims nach einem herkömmlichen Verfahren, wird das Glykosid-spaltende Enzym **nach** der Abtrennung des Pflanzenölschleims diesem zugesetzt, da ein solcher Pflanzenölschleim noch nicht mit erfindungsgemäßen Enzymen in Kontakt gebracht worden ist. Mit dieser Vorgehensweise kann folglich aus Pflanzenölschleim sowohl weiteres Öl als auch entöltes Lecithin gewonnen werden.

Beiden Alternativen gemeinsam sind die Verfahrensschritte des in-Kontakt-Bringens der Ausgangsmaterialien mit dem Glykosid-spaltenden Enzym und die anschließende Trennung in eine wässrige und eine ölige Phase; oder verkürzt ausgedrückt: die Gewinnung von lecithinfreiem Öl und ölfreiem Lecithin durch Trennung mittels Enzymen.

Es ist bekannt, dass der Großteil der Viskosität bzw. der Schleimstoffe in pflanzlichen Ölen durch Phospholipide hervorgerufen wird. Diese Viskosität wird in der Regel durch das Vorhandensein lamillare Phasen induziert. Im Vergleich zu den Phospholipiden stellen die restlichen Stoffe, welche viskositätserhöhend wirken bzw. den schleimigen Charakter der Öle verursachen, nur einen geringen Anteil dar. Vor diesem Hintergrund wurde nunmehr unerwartet gefunden, dass das Enzym Amylase, wenn es ausschließlich eingesetzt wird, die Viskosität der Pflanzenöle deutlich herabsetzt, indem weniger Öl in der Schleimphase ist und die Abtrennung einer wässrigen Schleimphase ermöglicht. Somit stellt dieses Verfahren eine Alternative zu den enzymatischen Entschleimungsverfahren des Standes der Technik dar. Das erfindungsgemäße Verfahren ist insbesondere für die Gewinnung von Lecithin bzw. Phospholipiden aus Pflanzenölen geeignet, da die erfindungsgemäßen Glykosid-spaltenden Enzyme die Phospholipide chemisch unverändert lassen.

Ohne sich an eine bestimmte Theorie zu binden, wurde gefunden, dass durch den Einsatz von Glykosidasen Glykolipide aufgespalten werden können. Die Klasse der Glykolipide umfasst eine Vielzahl von Verbindungen, wobei in dem Pflanzenölschleim bzw. in pflanzlichen Phospholipiden vor allen Dingen Sterylglykoside, Cerebroside und Galactosyllipide enthalten sind (Selmair, P.L., Koehler, P. in Molecular Structure and Baking Performance of Individual Glycolipid Classes from Lecithins, J. Agric. Food Chem. 2009, 57, 5597-5609). So zeigte sich beispielsweise, dass entfettetes Sojalecithin bis zu 10 Gew.-% Glykolipide enthält.

Eine weitere mögliche Erklärung des Mechanismus von Glykosidasen besteht im enzymatischen Abbau von oligomeren oder polymeren Kohlenhydraten, wie z.B. von Zellwandpektinen und Polysacchariden, ohne damit jedoch eine wissenschaftliche Aussage zu treffen.

Die Tatsache, dass eine Erhöhung der Ölausbeute mit der erfindungsgemäßen Enzymformulierung insbesondere dann erreicht wird, wenn man α-glykosidische Enzyme wie Amylase einsetzt, spricht eher für den zuletzt genannten Mechanismus der Wirkung der erfindungsgemäßen Enzyme bei der Ölentschleimung. In den am häufigsten in Pflanzenölen vorkommenden Glykolipiden, nämlich der Sterylglykoside und Galactosyl- und Digalactosysldiacylglycerole sind die Zuckerreste in der Regel über ßglykosidische Bindungen mit den Lipidresten verknüpft.

Schließlich könnte die Wirkung der vorliegenden Erfindung zum Teil durch die Entfernung von oligomeren Kohlenhydraten, wie zum Beispiel von Stachyose und Raffinose, erklärt werden. Stachyose ist ein Tetrasaccharid aus Saccharose (Glucose + Fructose) und zwei Galactosemolekülen, welche zur Familie der Raffinosen gehört. Charakteristisch für Stachyose sind (1→6)-α und (1→2)-β-glykosidische Bindungen. Raffinose ist ein Trisaccharid aus Galactose, Glucose und Fructose, wobei zwischen Galactose und Glucose eine (1α→6)- und zwischen Glucose und Fructose eine (1α→2β)-glykosidische Bindung vorliegt. Beide Arten von glykosidischen Bindungen werden vermutlich durch die erfindungsgemäß eingesetzten Enzyme, wie z.B. Amylasen, gespalten. Insgesamt wird erfindungsgemäß die Wirkung erzielt, dass weniger Öl in der abzutrennenden wässrigen Phase emulgiert und somit die Ölausbeute erhöht wird.

In einer weiteren bevorzugten Ausführungsform wird die Enzymaktivität der Glykosidasen im Bereich von 0,01 bis 6 units/g Öl, bevorzugt 0,1 bis 3 units/g Öl und besonders bevorzugt im Bereich von 0,2 bis 2,5 units/g Öl gewählt und am meisten bevorzugt im Bereich von 0,3 bis 1 units/g Öl. (Unit: Internationale Einheit für Enzymaktivität; 1 Unit entspricht dem Substratumsatz von 1 µmol/min).

Die Enzyme können dabei beispielsweise gefriergetrocknet und in Wasser oder entsprechendem Enzympuffer gelöst verwendet werden. Als Beispiel können Citratpuffer 0,01 - 0,25 M, pH 3,8-7,5, oder Acetatpuffer 0,01 - 0,25 M, pH 3,8-7,5 bevorzugt, genannt werden. In einer bevorzugten Ausführungsform werden die Enzyme in Wasser oder Enzympuffer aufgenommen und dem Rohöl zugesetzt. Um eine bessere Löslichkeit der Enzyme - insbesondere in den Phospholipide enthaltenden Mischungen - zu erreichen, ist auch der Zusatz von organischen Lösungsmitteln möglich. Diese finden Anwendung z. B. in der Auftrennung der Phospholipide. Bevorzugt verwendet werden unpolare organische Lösungsmittel wie z.B. Hexan oder Aceton oder Mischungen, bevorzugt in einer Menge von 1 bis 30 Gew.-% (Beispiele möglicher Lösungsmittel sind beschrieben in der EP 1531182 A2).

In einer weiteren bevorzugten Ausführungsform werden eines oder mehrere der verwendeten Enzyme in geträgerter Form eingesetzt. Im Rahmen der vorliegenden Erfindung bevorzugte Trägermaterialien sind anorganische Trägermaterialien wie z.B. Kieselgele, Fällungskieselsäuren, Silikate oder Alumosilikate und organische Trägermaterialien wie z. B. Methacrylate oder Ionentauscherharze. Der Vorteil des geträgerten Enzyms liegt in der leichteren Abtrennbarkeit des Enzyms und/oder der Wiederverwertbarkeit des Enzyms.

Es wurde überraschend gefunden, dass die erfindungsgemäßen glykosid-spaltenden Enzyme effektiv das Schleimvolumen und die Emulgierbarkeit von Pflanzenöl in wässrigen Phasen verringern. Dabei kann die erfindungsgemäße Verfahren besonders vorteilhaft für die Entschleimung von rohem Pflanzenöl oder auch zur Aufbereitung der Schleimphase eingesetzt werden. Die Schleimphase kann dabei beispielsweise durch ein herkömmliches Entschleimungsverfahren oder durch das erfindungsgemäße Verfahren, wenn es zur Entschleimung von rohem Pflanzenöl eingesetzt wird, erhalten werden.

Erstaunlicherweise wurde hierbei gefunden, dass es durch den Zusatz der Enzyme gelingt, die Reaktionsgeschwindigkeit bei der Entschleimung zu erhöhen, das Schleimvolumen zu erniedrigen und/oder die Abtrennbarkeit der gebildeten Schleimphase zu verbessern.

Das "in-Kontakt-Bringen" kann im Rahmen des erfindungsgemäßen Verfahrens auf jede Weise erfolgen, die dem Fachmann als für den erfindungsgemäßen Zweck geeignet bekannt ist. Bevorzugte Arten des in-Kontakt-Bringens ist dabei ein Vermischen des Rohöls und des Glykosid-spaltenden Enzyms.

Nach dem Mischen des Rohöls mit dem Enzym wird die Mischung aus Rohöl und Enzym bevorzugt gerührt und somit werden die Bestandteile miteinander in Kontakt gebracht. Besonders bevorzugt wird mit einem Flügelrührer bei 200 bis 800 U/min, bevorzugt 250 bis 600 U/min und am meisten bevorzugt bei 300 bis 500 U/min gerührt.

Die Temperatur der Mischung liegt während des in-Kontakt-Bringens bevorzugt im Bereich von 15 bis 99 °C, bevorzugter im Bereich von 20 bis 95 °C, weiter bevorzugt von 30 bis 80 °C, ebenfalls bevorzugt von 35 bis 80 °C, und besonders bevorzugt 37 bis 78 °C. Dabei soll gemäß einer Ausführungsform die Temperatur der Mischung immer so gewählt werden, dass die Denaturierungstemperatur der Enzyme nicht überschritten wird, bevorzugt liegt die Temperatur der Mischung mindestens 5 °C unter der Denaturierungstemperatur der Enzyme bzw. der niedrigsten Denaturierungstemperatur der Enzyme. Wobei bei Einsatz von Enzymen, die aus thermophilen Organismen isoliert wurden, grundsätzlich höhere Temperaturen zu bevorzugen sind. Werden im Rahmen der vorliegenden Erfindung ein oder mehrere thermostabile Enzyme eingesetzt, so liegt die Prozesstemperatur bevorzugt im Bereich von 60 bis 120 °C, bevorzugter im Bereich von 80 bis 100 °C. Die Verwendung thermostabiler Enzyme hat den Vorteil, dass somit eine erhöhte Verfahrenstemperatur gewählt werden kann, wodurch die Viskosität des Pflanzenöls verringert wird und das Verfahren insgesamt verkürzt werden kann - auch aufgrund einer erhöhten Reaktionsgeschwindigkeit der Enzyme. Des Weiteren erübrigt sich im Falle einer Vorbehandlung, die vorteilhaft ebenfalls bei erhöhten Temperaturen durchgeführt wird, ein anschließendes Abkühlen unterhalb einer niedrigeren Denaturierungstemperatur der eingesetzten Enzyme. Insgesamt führt die Verwendung thermostabiler Enzyme damit zur einer Verfahrensverkürzung und Kostensenkung.

Je nach Anwendung des Lecithins ist es bevorzugt, das im abgetrennten Lecithin enthaltene Enzym zu denaturieren, welche Maßnahme durch Erhitzen des Lecithins für 0,5 bis 10 Minuten auf 80 bis 100 °C Grad erfolgt, je nach eingesetztem Enzym. Im Falle der Verwendung von thermostabilen Enzymen ist darauf zu achten, dass das Lecithin durch den Denaturierungsvorgang thermisch nicht zu stark belastet wird, da es sonst unansehnlich wird bzw. sich verfärbt und z.B. für Lebensmittel-Anwendungen nicht mehr in Betracht kommt.

Die Dauer des in-Kontakt-Bringens liegt dabei bevorzugt im Bereich von 1 Minute bis 12 Stunden, bevorzugter von 5 Minuten bis 10 Stunden, weiter bevorzugt von 10 Minuten bis 3 Stunden.

Der pH Wert der Mischung liegt während des in-Kontakt-Bringens bevorzugt im Bereich von pH 3 bis pH 8, besonders bevorzugt im Bereich von pH 3,5 bis pH 7,5.

Das Abtrennen der Schleimstoffe gemäß Schritt b) des erfindungsgemäßen Verfahrens kann auf jede Weise erfolgen, die dem Fachmann als für den erfindungsgemäßen Zweck geeignet bekannt ist. Bevorzugt erfolgt die Abtrennung jedoch über Zentrifugation oder Filtration, wobei Zentrifugation bevorzugt ist. Bei der Zentrifugation erfolgt eine Phasentrennung der Mischung, sodass das behandelte Pflanzenöl, die Schleimstoffe und die Enzymzusammensetzung in separaten Phasen vorliegen, die leicht voneinander getrennt werden können.

In einer bevorzugten Ausführungsform wird dabei die Phase enthaltend die Schleimstoffe sowie die Phase, welche das Enzym für das erfindungsgemäße Verfahren enthält, von dem behandelten Öl abgetrennt. Besonders bevorzugt ist es dabei, wenn gleichzeitig mit den Schleimstoffen das Enzym abgetrennt wird.

Eine weitere bevorzugte Ausführungsform der vorliegenden Erfindung betrifft zudem ein Verfahren wie vorstehend beschrieben, weiter umfassend den Schritt
c) erneutes in-Kontakt-Bringen der Triglyceride gemäß Schritt b) mit der Enzymkomponente.

Das in-Kontakt-Bringen erfolgt dabei bevorzugt unter den gleichen Bedingungen wie vorstehend für Schritt a) des erfindungsgemäßen Verfahrens beschrieben. Dabei werden in einer besonders bevorzugten Ausführungsform die Enzyme vor dem erneuten in-Kontakt-Bringen einer Regeneration oder Reinigung unterzogen.

In einer besonders bevorzugten Ausführungsform wird das rohe Pflanzenöl vor dem in-Kontakt-Bringen gemäß Schritt a) des erfindungsgemäßen Verfahrens mit Wasser und/oder Säure in Kontakt gebracht. Bevorzugte Säuren sind dabei Calcium- und Magnesium-komplexierende Säuren allein oder in Kombination, wie z. B. Zitronensäure und Phosphorsäure.

In einer weiteren bevorzugten Ausführungsform des erfindungsgemäßen Verfahrens wird vor Schritt a) des Verfahrens eine sogenannte **Vorkonditionierung** durchgeführt indem das rohe Öl in einem eigenen Verfahrensschritt mit einer Menge von 200 - 2000 ppm an organischer Säure, bevorzugt Zitronensäure, vermischt wird. Die Temperatur der Mischung wird hierbei bevorzugt auf 35 bis 90 °C eingestellt, besonders bevorzugt 48°C oder 80°C. Nach einer Reaktionszeit von 5 Minuten bis 2 Stunden, bevorzugt 15 Minuten bis 1 Stunde wird die Mischung durch Zugabe einer stöchiometrischen Menge Lauge, bevorzugt Natronlauge in einer Menge von bevorzugt 0,5 bis 2 Mol/l, besonders bevorzugt 1 Mol/l auf einen pH von 4-5 eingestellt. Die wässrige Phase bzw. die Salzlösung werden nicht von der Ölphase abgetrennt, sondern es folgt die Ausführung des Schrittes a) des erfindungsgemäßen Verfahrens.

In einer bevorzugten Ausführungsform des erfindungsgemäßen Verfahrens erfolgt vor Schritt a) ein in-Kontakt-Bringen des Rohöles mit Wasser bei einer Temperatur zwischen 30°C bis 90°C für 5 bis 240 Minuten, bevorzugt 10 bis 60 Minuten, wobei eine Temperatur von 35 bis 90 °C bevorzugt und eine Temperatur von 40 bis 90 °C besonders bevorzugt ist. In einer weiteren möglichen Ausführungsform wird die Temperatur vor Zugabe des Enzyms auf eine Temperatur gebracht, die optimal für das eingesetzte Enzym ist. Temperaturen zwischen 35 bis 80 °C, besser zwischen 40 bis 78 °C sind geeignet, wobei durch Einsatz von Enzymen aus thermophilen Organismen, also besonders temperaturstabilen Enzymen, ein Einsatz bei 80 bis 100 °C möglich ist, so dass zwischen dem in-Kontakt-Bringen des rohen Pflanzenöls mit Wasser und dem in-Kontakt-Bringen mit dem Enzym des erfindungsgemäßen Verfahrens keine Temperaturabsenkung erfolgen muss. In einer weiteren möglichen Ausführungsform wird anschließend die wässrige Phase, z.B. durch Zentrifugation, abgetrennt.

Des Weiteren wird in einer bevorzugten Ausführungsform das Rohöl **vorentschleimt.** Das in-Kontakt-Bringen des rohen Pflanzenöles mit Wasser oder wässriger Säure, insbesondere Zitronensäure oder Phosphorsäure, erfolgt im Rahmen des erfindungsgemäßen Verfahrens bevorzugt bei einer Temperatur zwischen 30°C bis 90°C für 5 bis 240 Minuten, bevorzugt 10 bis 120 Minuten, wobei eine Temperatur von 35 bis 90 °C bevorzugt und eine Temperatur von 40 bis 90 °C besonders bevorzugt ist. In einer weiteren möglichen Ausführungsform wird anschließend die säurehaltige oder wässrige Phase z. B. durch Zentrifugation abgetrennt. In einer bevorzugten Ausführungsform wird nach der Säurebehandlung ein Neutralisationsschritt mit einer entsprechenden Base erfolgen, um einen pH-Wert von 3,5 bis 8,0, bevorzugt von 4 bis 7 zu erreichen. Anschließend kann das Öl von den erhaltenen Schleimstoffen durch z. B. Zentrifugation oder Filtration abgetrennt werden.

Vor Zusatz der Enzyme wird die Reaktionstemperatur vorzugsweise so eingestellt, dass sie den optimalen Temperaturbereich des Enzyms nicht übersteigt, um ein Denaturieren des Enzyms zu verhindern. Temperaturen zwischen 35 bis 80 °C, besser zwischen 40 bis 78 °C sind geeignet, wobei durch Einsatz von Enzymen aus thermophilen Organismen, also besonders temperaturstabilen Enzymen, ein Einsatz bei 80 bis 100 °C möglich ist, so dass zwischen dem in-Kontakt-Bringen des rohen Pflanzenöls mit Wasser und/oder Säure und dem in-Kontakt-Bringen mit dem Glykosid-spaltenden Enzym keine Temperaturabsenkung erfolgen muss. Eine Erhöhung der Temperaturstabilität kann auch durch eine Immobilisierung der Enzyme der Enzymkomponenten erreicht werden. Da viele Enzyme eine gewisse Toleranz gegenüber organischen Lösungsmitteln aufweisen (Faber, K., Biotransformations in Organic Chemistry (2001), Springer-Verlag, Heidelberg), können im Rahmen der vorliegenden Erfindung auch dementsprechend vorbehandelte Öle oder Schleime mit den Enzymen behandelt werden.

In besonders bevorzugten Ausführungsformen, die den Rahmen der vorliegenden Erfindung in keiner Weise beschränken, umfasst das Verfahren der vorliegenden Erfindung die Schritte

### Allgemeine Ausführungsform 1)

a) in-Kontakt-Bringen der Triglyceride, vorzugsweise ausgewählt aus Sojaöl und/oder Rapsöl und/oder Palmöl, mit einer Zusammensetzung, umfassend eine Enzymkomponente mit mindestens einem Glykosid-spaltenden Enzym, vorzugsweise einem Enzym, das alphaglykosidische Bindungen spaltet, insbesondere einem Enzym, das alpha(1-4)-glykosidische Bindungen spaltet;
b) Abtrennen der Schleimstoffe von den Triglyceriden durch Zentrifugation.

### Allgemeine Ausführungsform 2)

Gemäß der allgemeinen Ausführungsform 2) wird - anstelle des rohen Pflanzenöls - die durch ein herkömmliches Entschleimungs-Verfahren oder durch das erfindungsgemäße Verfahren abgetrennte Schleimphase mit dem Glykosid-spaltenden Enzym "in-Kontakt-gebracht". Das Verfahren erfolgt bevorzugt gemäß der Ausführungsform 1). Dieses Verfahren ermöglicht beispielsweise die Rückgewinnung von Öl aus der Schleimphase, welches Öl in der Schleimphase enthalten war und von dieser abgetrennt wurde; es führt damit zu einer indirekten Erhöhung der Ölausbeute.

Wenn die Gewinnung des Pflanzenölschleims nach einem herkömmlichen Verfahren, z.B. mit Wasser oder mit wässriger Säurelösung, erfolgt ist, kann das erfindungsgemäße Enzym **nach** Abtrennung der (Lecithin-haltigen) Schleimphase zu dem Pflanzenölschleim gegeben werden, um weiteres Öl aus dem Pflanzenölschleim zu extrahieren. In diesem Fall bezieht sich der Ausdruck "nach Abtrennung der (Lecithin-haltigen) Schleimphase" nicht auf den Schritt b) bzw. b2) des erfindungsgemäßen Verfahrens.

Im Gegensatz zur allgemeinen Ausführungsform 2) wird in der allgemeinen Ausführungsform 1) das erfindungsgemäße Enzym **vor** der Abtrennung der Schleimphase vom Öl (Schritt b1) zugegeben. Dieser Umstand ist durch die Reihenfolge der Schritte a) und b1) festgelegt.

Die Verfahrensschritte in den Ausführungsformen 1) und 2) sind identisch: nämlich a) Kontaktieren des Ausgangsmaterials, seien es (rohe) Triglyceride oder (unvollständig entölter) Pflanzenölschleim, mit dem erfindungsgemäßen Enzym, und Trennen der Mischung in eine wässrige Schleimphase und eine Triglycerid-haltige Ölphase im Falle von Triglyceriden nach Schritt b1) bzw. Trennen in eine wässrige (Lecithin-haltige) Phase und eine Ölphase im Falle von Pflanzenölschleim aus Ausgangsmaterial nach Schritt b2). Ebenso erfolgt die Durchführung des Verfahrens nach beiden Ausführungsformen 1) und 2) mit den gleichen Vorrichtungen und nach den gleichen Prinzipien.Mit dem erfindungsgemäßen Verfahren ist es möglich, das Schleimvolumen des Öls abzusenken, ohne dass Phospholipid-spaltende Enzyme eingesetzt werden.

### Methoden

### Bestimmung der Ölausbeute, des Ölgehalts in der Schleimphase und des Schleimvolumens

Die Bestimmung der Ölausbeute, des Ölgehalts in der Schleimphase und des Schleimvolumens sind durch die Bestimmung des Schleimvolumens nach standardisiertem Verfahren, wie sie auch in PCT/EP 2013/053 199 beschrieben sind, detektierbar. Weiterhin kann durch Soxhlet-Extraktion des isolierten Schleims der Ölgehalt des Schleims separat gemäß DIN ISO 659 bestimmt werden.

### Bestimmung der Phospholipase-Aktivität

Um eine Phospholipase- oder Acyltransferase-Aktivität bei dem erfindungsgemäßen Verfahren auszuschließen, wurde der Gehalt an freien Fettsäuren während des Entschleimungsverfahrens im Öl untersucht. Dies erfolgt nach Modifikation der Referenzmethode N.G.D. C10, der American Oil Chemistry Society (AOCS) Ca 5a-40.

Zur Bestimmung der freien Fettsäuren wird ein Foodlab-Gerät der Firma cdR (Italien) verwendet, das ein selbstständiges, kompaktes Analysengerät mit einem eingebauten Spektralphotometer darstellt; es besteht aus einem temperierten Inkubationsblock mit 12 Zellen für Küvetten und 3 unabhängigen Messzellen mit jeweils 2 Lichtstrahlen von unterschiedlicher Wellenlänge.

Nach dem Einschalten des Foodlab-Geräts zur Photometrischen Bestimmung des Anteils freier Fettsäuren (FFA), werden die gebrauchsfertigen Messküvetten der Firma CDR auf 37°C vorgewärmt, dann erfolgen die Auswahl der Methode zur FFA-Bestimmung im Menü und die Bestimmung des Blindwerts der Küvette. Anschließend wird das erforderliche Volumen Pflanzenöl in die Lösung der Messküvette, bestehend aus einem Mix diverser Alkohole, KOH und Phenolphthalein-Derivaten, einpipettiert. Je nach FFA-Gehalt werden für Sojaöl üblicherweise 2,5 µL und für Rapsöl 1 µL Probe verwendet. Das aus der Pflanzenölprobe aufgenommene Volumen wird ein Mal verworfen um die Pipette zu spülen, dann wird erneut Probe aufgenommen und in die fertige Messlösung pipettiert. Die Pipette wird danach exakt zehnmal mit der Messlösung gespült, um das Volumen der Ölprobe so wenig wie möglich zu verfälschen. Daraufhin wird die Küvette zehnmal per Hand geschwenkt. Die Fettsäuren in der Probe (bei pH <7,0) reagieren mit einem chromogenen Anteil und bilden einen Farbkomplex, dessen Intensität anschließend bei 630 nm in der Messzelle des Geräts bestimmt wird. Er wird vom Gerät in Prozent von Ölsäure angezeigt und ist proportional der Gesamtsäurekonzentration in der Probe.

Während der enzymatischen Entschleimung über 4 Stunden beträgt im Allgemeinen die (relative) Steigerung der Konzentration der freien Fettsäuren, ausgedrückt als Ölsäure und bezogen auf die Gesamtmenge der gesamten Fettsäuren, nicht mehr als 10 %, vorzugsweise nicht mehr als 8 %. Die Bestimmung wird nach der Modifikation der Referenzmethode N.G.D. C10, AOCS Ca 5a-40 durchgeführt.

Die Erhöhung der Konzentration an freien Fettsäuren beträgt während der enzymatischen Entschleimung z. B. für ein Sojaöl nicht mehr als eine Steigerung von z. B. 0,22 Gew.-% freie Fettsäuren auf 0,24 Gew.-% freie Fettsäuren, bestimmt als freie Ölsäure und bezogen auf die Gesamtmasse der Fettsäuren, bei einem pH-Wert < 7 und bestimmt nach Modifikation der Referenzmethode N.G.D. C10, AOCS Ca 5a-40; (siehe Tabelle 1)

Im Vergleich zu den erfindungsgemäßen Enzymen ist die Steigerung der Konzentration der freien Fettsäuren, gemessen nach derselben Methode, bei Zugabe eines Phospholipid-spaltenden Enzyms, wie z.B. der Phospholipase A1 (PLA 1), in Tabelle 1 dargestellt. Im Laufe der Reaktion erhöht sich die Konzentration der FFA von 0,15 Gew.-% nach 10 min Reaktionszeit auf 0,34 Gew.-% nach 240 min Reaktionszeit, so dass eine relative Zunahme der FFA-Konzentration um 126 % vorliegt.

Ähnliche Verhältnisse liegen bei der enzymatischen Entschleimung von z.B. einem Rapsöl vor (siehe Tabelle 2). Die erfindungsgemäße Amylase aus Aspergillus erhöht die Konzentration an freien Fettsäuren von 1,69 Gew.-% nach 10 min auf 1,71 Gew.-% nach 240 min nur um relativ 1,2 %. Ebenso steigert die Amylase PET die Konzentration an freien Fettsäuren von 1,76 Gew.-% nach 10 min auf 1,79 Gew.-% nach 180 min, so dass eine relative Zunahme der FFA-Konzentration von 1,7 % vorliegt.

Im Gegensatz dazu steigert das Phospholipid-spaltende Enzym PLA 1 die Konzentration an freien Fettsäuren von z.B. 1,76 Gew.-% nach 10 min auf 2,22 Gew.-% nach 240 min, so dass eine relative Zunahme der FFA-Konzentration um 26 % vorliegt.

**Tabelle 1: Sojaöl: Ergebnisse der FFA-Messungen in Gew.-% während der enzymatischen Ölentschleimung und Vergleich mit einem Glykosid-spaltenden Enzym - Phospholipase A 1 (PLA 1)**

| **Mittel** | **10 min** | **180 min** | **240 min** |
|---|---|---|---|
| FFA [%] H3Cit (Zitronensäure) | 0,26 | 0,25 | 0,23 |
| FFA [%] α-Amylase Bacillus sp. | 0,22 | 0,24 | 0,22 |
| FFA [%] PLA 1 | 0,15 | 0,31 | 0,34 |

**Tabelle 2: Rapsöl: Ergebnisse der FFA-Messungen in Gew.-% während der enzymatischen Ölentschleimung und Vergleich mit einem Glykosid-spaltenden Enzym - Phospholipase A 1 (PLA 1)**

| **Mittel** | **10 min** | **180 min** | **240 min** |
|---|---|---|---|
| FFA [%] H3Cit (Zitronensäure) | 1,73 | 1,68 | 1,72 |
| FFA [%] α-Amylase Aspergillus | 1,69 | 1,65 | 1,71 |
| FFA [%] α-Amylase PET | 1,76 | 1,79 | 1,73 |
| FFA [%] PLA 1 | 1,76 | 1,99 | 2,22 |

Die Einheiten der Werte in den Tabellen 1 und 2 stellen Gew.-% dar und geben den Anteil der freien Fettsäuren, berechnet als Ölsäure, im Verhältnis zu den gesamten Fettsäuren an. Die Werte werden nach der Modifikation der Referenzmethode N.G.D. C10, AOCS Ca 5a-40 bestimmt.

### Variante 1:

Die zu behandelnde Menge Rohöl 400 bis 600 g, wird in einen Duran Reaktor DN120 1000 mL eingefüllt und Proben für die Analytik werden abgenommen. Das Öl im Duranreaktor wird mithilfe einer Heizplatte auf eine Temperatur von 35 bis 90 °C, insbesondere 48 °C oder insbesondere 80°C, aufgeheizt. Nachdem die Temperatur erreicht ist, wird mit der Vorkonditionierung begonnen. Dafür wird eine definierte, von der Ölmenge abhängige Menge verdünnter Zitronensäure (z. B. 450 ppm, 1,372 mL), zum Öl zudosiert. Anschließend wird die Mischung mit einem Ultraturrax für 1 Minute durchmischt. Als Alternative wird unter Rühren bei etwa 600 rpm, 1 Stunde inkubiert um die Reaktion der Säure abzuwarten. Anschließend wird eine definierte Menge Natronlauge (4 Mol/L, Restmenge zu 3 % v/v, abzüglich Wasser aus Säurezugabe und Enzymzugabe) zugegeben und es wird weitere 10 Minuten unter Rühren inkubiert. Bei der Vorbehandlung bei 80°C wird vor Zugabe des Enzyms die Mischung auf z. B 50°C heruntergekühlt. Dann erfolgt die Zugabe des Enzyms, der Enzym-Mischung oder des Immobilisats, vorzugsweise gelöst in Puffer. Das Enzym wird untergerührt, wofür die Rührerdrehzahl kurzzeitig erhöht werden kann (1 Minute auf 900 rpm), anschließend wird bei niedrigerer Drehzahl weitergerührt. Am Ende der Reaktion wird die Ölphase von der Schleimphase durch Zentrifugation getrennt, und der Restölgehalt der Schleimphase nach Soxhlet-Extraktion bestimmt.

### Variante 2:

In einer weiteren Durchführung werden Glykosid-spaltenden Enzyme alleine oder in einer geeigneten Kombination als freie Enzyme oder immobilisierte Enzyme zusammen mit einer wässrigen Phase 0,05 bis 5 % w/v, dem Rohöl zugesetzt. Die Emulsion, bestehend aus Wasser, Enzym, eventuell Enzymträgern und Öl, wird durchmischt. Idealerweise wird die Reaktion temperiert zwischen 30 bis 80 °C, besser zwischen 40 bis 78 °C durchgeführt. Anschließend wird die Phasentrennung abgewartet, die Feststoffe setzen sich ab oder können nach einem dem Fachmann bekannten Standardverfahren z. B. über Zentrifugation oder Filtration entfernt werden. Als Nachbehandlung kann das Öl mit verdünnter Säure (z. B. Zitronensäure) oder Lauge nach einem dem Fachmann als "Degumming" bekannten Verfahren restentschleimt werden.

### Variante 3:

In einer weiteren Durchführung wird Ölschleim mit Enzymen behandelt. Dem Ölschleim, welcher nach einem dem Fachmann als "degumming" bekannten Verfahren erhalten wird, werden Glykosid-spaltende Enzyme zugesetzt. Diese können sich gelöst in einer wässrigen Phase oder suspendiert in einem organischen Lösungsmittel befinden. Der Ansatz wird idealerweise auf eine Temperatur zwischen 20 bis 70 °C temperiert, besser auf eine Temperatur zwischen 35 bis 60 °C. Der Ansatz wird durchmischt bis der Prozess abgeschlossen ist. Dies kann durch Viskositätsmessungen überprüft werden oder visuell, durch Auflösen der ansonsten festen Schleimphase. Durch Zentrifugation lässt sich eine Phasenseparation erreichen, die einzelnen Phasen können abgetrennt werden. In der Regel besteht die obere Phase aus dem gewonnenen Öl, die mittlere Phase aus den Phospholipiden und die untere Phase ist eine wässrige Phase und enthält die Enzyme. Durch Wiederverwendung der wässrigen Phase lassen sich die Enzyme recyceln und wiederverwenden. Je nach Gehalt zweiwertiger Ionen muss das Öl oder die das Enzym enthaltende Wasserphase durch Zusatz von Komplexierungsmitteln vor der weiteren Verwendung von den Ionen bereinigt werden.

### Variante 4:

In einer weiteren Durchführung wird das Rohöl auf eine hohe Temperatur gebracht, vornehmlich 70 bis 100 °C, genauer 75 bis 85 °C. Das Rohöl wird nach dem oben beschriebenen Verfahren mit Säure und Lauge konditioniert, die Temperatur wird beibehalten und es werden thermostabile Enzyme zusetzt. Im Weiteren wird wie bereits beschrieben fortgefahren. Das Enzym wird untergerührt, wofür die Rührerdrehzahl kurzzeitig erhöht werden kann (z. B. 1 Minute auf 900 rpm), anschließend wird bei 600 rpm weitergerührt, bis die Reaktion beendet ist. Die Abtrennung des Ölschleims kann wie vorhergehend beschrieben erfolgen.

### Variante 5:

In einer weiteren Durchführung wird das Rohöl auf eine hohe Temperatur gebracht, vornehmlich 70 bis 100 °C, genauer 75 bis 85 °C. Es werden thermostabile Glykosid-spaltenden Enzyme alleine oder in einer geeigneten Kombination als freie Enzyme oder immobilisierte Enzyme zusammen mit einer wässrigen Phase 0,05 bis 5 % w/v, dem Rohöl zugesetzt. Die Emulsion, bestehend aus Wasser, Enzym, eventuell Enzymträgern und Öl, wird durchmischt. Im Weiteren wird wie bereits beschrieben fortgefahren. Das Enzym wird untergerührt, wofür die Rührerdrehzahl kurzzeitig erhöht werden kann (z. B. 1 Minute auf 900 rpm), anschließend wird bei 600 rpm weitergerührt, bis die Reaktion beendet ist. Die Abtrennung des Ölschleims kann wie vorhergehend beschrieben erfolgen.

### Beispiele:

Die Erfindung wird im Weiteren an Hand von Beispielen näher erläutert. Es wird hierbei betont, dass die Beispiele lediglich veranschaulichenden Charakter besitzen und besonders bevorzugte Ausführungsformen der vorliegenden Erfindung veranschaulichen. Die Beispiele beschränken den Rahmen der vorliegenden Erfindung nicht.

### Beispiel 1:

Gemäß Reaktionsvariante 1 wurde ein Sojaöl einer Vorkonditionierung mit Hilfe von wässriger Zitronensäure (1000 ppm) und wässriger Natronlauge (4 Mol/L) unterzogen (Gesamtwassergehalt der Reaktion: 3%). Als Vergleich wurde eben diese Vorkonditionierung mit Zugabe eines Enzyms, alpha Amylase aus dem Organismus aus dem Organismus *Bacillus spec.* (Sigma-Aldrich) durchgeführt (siehe Tabelle 1).

**Tabelle 3: Sojaöl: Gesamtölausbeute der Reaktionen aus Beispiel 1 nach Soxhlet-Extraktion der Schleimphase**

| **Mittel** | **Ölausbeute [%]** |
|---|---|
| H3Cit (Zitronensäure) | 97,1 |
| α-Amylase Bac. Sp. | 97,9 |

### Beispiel 2:

Gemäß Reaktionsvariante 1 wurde ein Rapsöl einer Vorkonditionierung mit Hilfe von wässriger Zitronensäure (1000 ppm) und wässriger Natronlauge (4 Mol/L) unterzogen (Gesamtwassergehalt der Reaktion: 3%). Als Vergleich wurde eben diese Vorkonditionierung mit Zugabe eines Enzyms Amylase PET aus dem Organismus *Bacillus subtilis* (ASA Spezialenzyme GmbH) oder einer α-Amylase aus *Aspergillus* oryzae (Sigma-Aldrich)durchgeführt (siehe Tabelle 2).

**Tabelle 4: Rapsöl: Gesamtölausbeute der Reaktionen aus Beispiel 2 nach Soxhlet-Extraktion der Schleimphase**

| **Mittel** | **Ölausbeute [%]** |
|---|---|
| H3Cit (Zitronensäure) | 96,2 |
| Amylase PET | 97,4 |
| **α** Amylase Aspergillus | 96,4 |

In den Tabellen 1 und 2 ist die Gesamtölausbeute der Reaktionen aus Beispiel 1 und 2 nach Soxhlet-Extraktion der Schleimphase dargestellt. Hierbei ist ersichtlich, dass die eingesetzten Glykosid-spaltenden Enzyme die Ölausbeute im Vergleich zum Standardprozess mit Zitronensäure erheblich steigern.

Es werden weltweit 43 Mio Tonnen Sojaöl hergestellt. Die Ausbeutesteigerung der Ölmenge von 97,1% beim Standardprozess auf 97,9% durch eine alpha-Amylase Bac. spec. würde bedeuten, dass 0,35 Mio Tonnen mehr Sojaöl hergestellt werden könnten.

Von Rapsöl werden weltweit ca. 23,5 Mio Tonnen hergestellt. Hier bedeutet die Ausbeutesteigerung der Ölmenge von 96,2% beim Standardprozess auf 97,4% durch die Amylase PET, dass ca. 0,3 Mio Tonnen mehr Rapsöl hergestellt werden könnten.

## Patentansprüche

1. Verfahren zur enzymatischen Entschleimung von Triglyceriden oder zur Verringerung des Ölgehalts im Pflanzenölschleim, der bei der Ölentschleimung anfällt, welches Verfahren folgende Schritte umfasst:
a) in-Kontakt-Bringen der Triglyceride oder des Pflanzenölschleims, der bei der Ölentschleimung anfällt, mit einer Zusammensetzung, die mindestens ein Glykosid-spaltendes Enzym umfasst, wobei das mindestens eine Glykosid-spaltende Enzym keine Phospholipase- und keine Acyltransferase-Aktivität aufweist bzw. keine Phospholipase und keine Acyltransferase in der Zusammensetzung vorhanden sind; und
b1) im Fall von Triglyceriden als Ausgangsmaterial: Abtrennen der Schleimstoffe von den Triglyceriden; oder
b2) im Fall von Pflanzenölschleim als Ausgangsmaterial: Auftrennung in eine wässrige, Lecithin-enthaltende Phase und eine Öl-haltige Phase.

2. Verfahren nach Anspruch 1, wobei das Triglycerid ein Pflanzenöl oder ein Gemisch aus einem pflanzlichen Öl und einem Öl tierischen Ursprungs ist.

3. Verfahren nach Anspruch 1, wobei das mindestens eine Glykosid-spaltende Enzym α(1-4)glykosidische, α(1-2)glykosidische, α(1-6)glykosidische,β(1-2)glykosidische, β(1-3)glykosidische, β(1-4)glykosidische und/oder β(1-6)glykosidische Bindungen spalten.

4. Verfahren nach Anspruch 1 oder 3, wobei das mindestens eine Glykosid-spaltende Enzym aus Amylasen, Amyloglucosidasen, Isoamylasen, Glucoamylasen, Glukosidasen, Galactosidasen, Glucanasen, Pullulanasen, Arabinasen, Laminaranasen, Pektolyasen, Mannanasen, Dextranasen, Pectinasen, Cellulasen, Cellobiasen, und Xylanasen ausgewählt wird.

5. Verfahren nach Anspruch 4, wobei die Amylase eine alpha-Amylase ist.

6. Verfahren nach Anspruch 5, wobei die alpha-Amylase aus Bacillus sp., Bacillus subtilis, Bacillus licheniformis, Bacillus megaterium, Bacillus amyloliquefaciens, Bacillus stearothermophilus, Pseudomonas aeroginosus, Pseudomonas fluorescens, Aspergillus oryzae, oder Aspergillus niger stammt.

7. Verfahren nach einem der vorhergehenden Ansprüche, wobei eines oder mehrere der eingesetzten Glykosid-spaltenden Enzyme in geträgerter Form vorliegen.

8. Verfahren nach einem der vorhergehenden Ansprüche, wobei Sojaöl, Rapsöl, Sonnenblumenöl, Olivenöl, Palmöl, Jatrophaöl, Leindotteröl oder Baumwollsaatöl als Öle eingesetzt werden.

9. Verfahren nach einem der vorstehenden Ansprüche, wobei anstelle des Pflanzenöls ein wässriger Pflanzenölschleim, der bei der Ölentschleimung von einem der Öle nach Anspruch 8 anfällt, eingesetzt wird.

10. Verwendung eines Glykosid-spaltenden Enzyms, wie in einem der Ansprüche 1 bis 9 definiert, zur Erhöhung der Ölausbeute bei der Ausführung einer wässrigen Öl-Entschleimung.

11. Verwendung nach Anspruch 10 zur Entölung von Lecithin.
